# EUROPÄISCHE PATENTSCHRIFT

(11) **EP 2 008 639 B1**
(45) Veröffentlichungstag und Bekanntmachung des Hinweises auf die Patenterteilung: **14.04.2021**
(21) Anmeldenummer: 08158352.8
(22) Anmeldetag: 16.06.2008
(51) Int. Cl.: A61K 8/06, A61Q 19/10, A61K 8/31, A61K 8/37, A61K 8/44, A61K 8/46, A61K 8/81, A61K 8/92

(54) **NEUE EMULSIONSFÖRMIGE DUSCHZUBEREITUNG**
NEW EMULSION-FORM SHOWER PREPARATION
NOUVELLE PRÉPARATION POUR DOUCHE SOUS FORME D'ÉMULSION

(30) Priorität: 20.06.2007 DE 102007029241
(43) Veröffentlichungstag der Anmeldung: 31.12.2008
(73) Patentinhaber: Beiersdorf Aktiengesellschaft, 20245 Hamburg (DE)
(72) Erfinder: Argembeaux, Horst, 21456 Wentorf (DE); Kohut, Michaela, 20255 Hamburg (DE); Ruppert, Stefan, 20259 Hamburg (DE); Counradi, Katrin, 22143 Hamburg (DE)

(56) Entgegenhaltungen:
- EP-A2- 1 291 001
- EP-A2- 1 470 813
- WO-A1-96/17591
- WO-A1-96/37595
- WO-A1-2005/030163
- WOODRUFF J: "VaMa Surfactants" SPC,, 1. November 2004 (2004-11-01), Seiten 1-4, XP007915282
- NN: "Carbopol (TM) Aqua SF-1 Polymer" The Lubrizol Corporation, Lubrizol Advanced Materials Inc., Cleveland Ohio; TDS-294, 1. Juli 2003 (2003-07-01), Seiten 1-9, XP002604527 Gefunden im Internet: URL:www.lubrizol.com/WorkArea//DownloadAss et.aspx?id=31978 [gefunden am 2010-10-08]

## Beschreibung

Die Erfindung betrifft emulsionsförmige Duschzubereitungen mit besonderem Schaum- und Anwendungsverhalten.

Zubereitungen, die zur Körperreinigung unter der Dusche verwendet werden, sind hinlänglich bekannt. Viele Zubereitungen stellen verdickte tensidhaltige wässrige Systeme dar, so genannte Duschgele. Auch emulsionsförmige Zubereitungen zur Körperreinigung unter der Dusche sind bekannt.

WO 2006/012465 offenbart eine Reinigungszubereitung mit rund 2.6 bis 3 Gew.-% einer wässrigen Acrylat-Copolymeremulsion, rund 7.7 bis 10.2 Gew.-% eines anionischen Tensides und rund 1.4 bis 1.7 Gew.-% eine amphoteren Tensides

EP 1 003 462 offenbart eine wässrige Zubereitung, deren ÖI- oder Emollienziengehalt den Gehalt an Tensiden übersteigen kann.

EP 346 097 offenbart ein flüssiges System enthaltend ein flüssiges Medium und eine Verdickermischung, die in dem flüssigen Medium dispergiert ist und ein Polymer vom Gummi-Typ und ein Acrylpolymer enthält, wobei jedes dieser Polymere auf besondere Weise unter Berücksichtigung u. a. seiner sigmoiden Kurve ausgewählt wird.

EP 662 816 offenbart Haarbehandlungsmittel mit Polymer und bestimmten Alkylpolyglucosiden.

EP 1 560 559 offenbart Haarreinigungszubereitungen mit reinigendem Tensid, 0.01 bis 0.5% eines Kationpolymer mit einer mittleren Ladungsdichte bei pH7 von 0.2 bis 1.0 meq/g, 0.01 bis 0.4% eines Kationpolymer mit einer mittleren Ladungsdichte bei pH7 von 1.3 bis 3.0 meq/g, wenigstens 40% Wasser und 0.1 bis 10% einzelner, dispergierter Tropfen eines wasserunlöslichen konditionierenden Öls mit einem mittleren Teilchendurchmesser von höchstens 4 µm, wobei die Kationpolymere im wesentlichen aus den selben Monomereinheiten und kationischen Gruppen bestehen.

EP 1 559 774 offenbart eine Reinigungszubereitung mit einem Tensidsystem aus wenigstens einem anionischen und einem amphotheren Tensid und einem Verdickersystem mit wenigstens zwei anionischen Polymeren mit mindestens einer hydrophoben Kette.

EP1291001 offenbart kosmetische Zusammensetzungen enthaltend vernetztes Copolymer auf Basis von Methacrylsäure und C1-C4 Alkylacrylat und ein Öl (synthetisch oder natürlich), unter anderem auch zur Reinigung und Pflege von Keratinstrukturen z.B. Haar oder Haut. Die in diesen Schriften offenbarten oder andere, im Markt befindliche Duschemulsionen schäumen entweder schlecht, lassen sich schlecht abspülen oder haben unter der Dusche ein sehr schmierig/glitschiges Hautgefühl, was der Verbraucher als nicht so angenehm empfindet.

Es hat sich nun für den Fachmann unerwartet herasgestellt, dass eine Körperreinigungszubereitung in Form einer Emulsion enthaltend wenigstens 1,8 Gew.-% amphothere oder zwitterionische Tenside und wenigstens 1 Gew.% Natriumcocoylglutamat und ein vernetztes Copolymer aus Methacrylsäure und C1 bis C4-Alkylacrylat, wobei der Gesamttensidgehalt 6 bis 25 Gew.-%; bevorzugt 8 bis 20 Gew.-%; besonders bevorzugt 12 bis 16 Gew.-% beträgt, den Nachteilen des Standes der Technik abhilft.

Durch den Einsatz dieses speziellen Gelbildners als Stabilisator und des darauf speziell abgestimmtes Tensidsystem kann eine stabile, gut schäumende und gut abspülbare Duschemulsion mit überlegender Sensorik (reichhaltig, aber nicht schmierig) erhalten werden.

Daher umfasst die Erfindung auch die Verwendung von wenigstens 1,8 Gew. Gew.-% amphotherer oder zwitterionischer Tenside und wenigstens 1 Gew.-% Natriumcocoylglutamat und einem vernetztem Copolymer aus Methacrylsäure und C1 bis C4-Alkylacrylat bei einem Gesamttensidgehalt von 6 bis 25 Gew.-% zur Verbesserung der Schaummenge von kosmetischen Reinigungsemulsionen sowie die Verwendung von wenigstens 1,8 Gew. Gew.-% amphotherer oder zwitterionischer Tenside und wenigstens 1 Gew.-% Natriumcocoylglutamat und einem vernetztem Copolymer aus Methacrylsäure und C1 bis C4-Alkylacrylat bei einem Gesamttensidgehalt von 6 bis 25 Gew.-% zur Verbesserung der Schaumqualität von kosmetischen Reinigungsemulsionen.

Bei all dem ist es bevorzugt, wenn das Verhältnis Natriumcocoylglutamat zu amphotheren oder zwitterionischen Tensiden 1,5:1 bis 12:1; bevorzugt 3:1 bis 10:1; besonders bevorzugt 5:1 bis 7:1 beträgt.

Weiter ist es bevorzugt, wenn der Methacrylsäureanteil im Copolymer 25 bis 70 Gew.-%, besonders bevorzugt 35 bis 65 Gew-.% beträgt. Weiter ist es bevorzugt, wenn der Anteil an Alkylacrylat im Copolymer 25 bis 75 Gew.-%, besonders bevorzugt 35 bis 65 Gew.-% beträgt. Weiter ist es bevorzugt, wenn als Alkylacrylat im Copolymer das Methyl, Ethyl- oder Buylacrylat, besonders bevorzugt das Ethylacrylat eingesetzt wird.

Weiter ist es bevorzugt, wenn das Copolymer unter Verwendung von 0,03 bis 1 Gew.-% mehrfach ethylenisch ungesättigtem Vernetzungsmittel vernetzt wird.

Weiter ist es bevorzugt, wenn das Copolymer in Konzentrationen von 1 bis 6 Gew.-%, bevorzugt 2 bis 4 Gew.-%, besonders bevorzugt 2,5 bis 3,5 Gew.-% eingesetzt wird.

Weiter ist es bevorzugt, wenn Öle in Konzentrationen von 5 bis 30 Gew.-%, bevorzugt 7 bis 20 Gew.-%, besonders bevorzugt 9 bis 11 Gew.-% eingesetzt werden.

Weiter ist es bevorzugt, wenn Öle in Form von natürlichen Triglyceriden eingesetzt werden, besonders bevorzugt in Anteilen von 10 - 50% bezogen auf den Ölanteil, ganz besonders bevorzugt 25 - 35%.

Weiter ist es bevorzugt, wenn die Tröpfchengröße der Emulsion zwischen 500 nm bis 2000 nm, besonders bevorzugt (d(0,5)=1000 nm liegt.

Weiter ist es bevorzugt, wenn die Tröpfchengröße der Emulsion eine inhomogene Verteilung aufweist. Weiter ist es bevorzugt, wenn die Tröpfchen der Emulsion in Form von Tröpfchenagglomeraten vorliegen.

Weiter ist es bevorzugt, wenn die Emulsion keine lamellare Phase aufweist.

Weiter ist es bevorzugt, wenn die Fliessgrenze der Emulsion 3 bis 30 Pa beträgt.

Fließgrenze im Sinne dieser Schrift ist die kritische Schubspannung der Fließkurve. Sie kann wie folgt ermittelt werden:
Gemessen wird die Fließkurve auf einem schubspannungsgesteuerten Rheometer bei 25°C ± 1°C mit 25 mm Platte/Platte Geometrie bei einem Spalt zwischen 0,8 mm und 1,2 mm, wobei strukturschonend befüllt wird. Es wird eine geeignete konstante Schubspannungszeitrampe vorgegeben und vor dem Test eine entsprechende Strukturerholungszeit eingehalten und die kritische Schubspannung im Maximum der Fließkurve angegeben.

Weiter ist es bevorzugt, wenn die Viskosität der Zubereitung1500 bis 7000 mPas beträgt. Die Messung der Viskosität im Sinne dieser Schrift erfolgt bei 25 °C und einer Scherrate von 100 1/s im Rheomat R 123, Messsystem 1, Messkörper 1.

Weiter ist es bevorzugt, wenn die Zubereitung zusätzlich PQ 7 enthält.

Weiter ist es bevorzugt, wenn die Zubereitung 0 bis 0,01 Gew.-% Fettalkoholethoxylate enthält.

Weiter ist es bevorzugt, wenn die Zubereitung 0 bis 0,01 Gew.-% Fettsäuren enthält.

Weiter ist es bevorzugt, wenn die Zubereitung 0 bis 0,01 Gew.-% Polysaccharide enthält.

Weiter ist es bevorzugt, wenn die Zubereitung neben dem vernetzten Copolymer aus Methacrylsäure und C1 bis C4-Alkylacrylat keinen weiteren Gelbildner enthält.

Weiter ist es bevorzugt, wenn die Zubereitung nicht mehr als ein kationisches Polymer enthält.

Es ist dabei von Vorteil, wenn zusätzlich ein Verdickungsmittel vom Typ PEG-Ether oder -Ester oder veresterter PEG-Ether in den Zubereitungen enthalten ist, bevorzugt PEG-200 Hydrogenated Glyceryl Palmate, PEG-120 Methyl Glucose Dioleate, PEG-90 Glyceryl Isostearate.

Von Vorteil ist es, wenn Konservierungsmittel enthalten sind, insbesondere DMDM Hydantoin.

### Beispiele

| | 1 | 2 | 3 |
|---|---|---|---|
| Paraffinöl | 4 | 6 | 9 |
| Sojaöl | 4 | 3 | 1 |
| Natrium Laurylethersulfat | 9 | 10 | 11 |
| Cocamidopropyl Betain | 2 | 2 | 2 |
| Sodium Cocoyl Glutamate | 2 | 2 | 1 |
| Acrylates Copolymer | 3,2 | 2,9 | 2,8 |
| Natriumhydroxid | - | - | q.s. |
| Polyquaternium-7 | 0,2 | 0,2 | 0,2 |
| Phenoxyethanol | 0,8 | 0,8 | 0,8 |
| Parabene | 0,7 | 0,7 | 0,7 |
| EDTA | 0,25 | 0,25 | 0,25 |
| Parfum | q.s. | q.s. | q.s. |
| Wasser | ad 100 | ad 100 | ad 100 |

Diese Zubereitungen sind stabile Duschemulsionen, die besonders viskositätsstabil sind, auch nach Lagerung bei 40°C über längere Zeiträume. Sie schäumen trotz des Ölgehalts gut und lassen sich sehr gut vom Körper abspülen. Ihre Sensorik ist sehr angenehm.

Die Konzentrationen in den Beispielen sind Aktivgehalte.

Folgende Handelsprodukte wurden eingesetzt:

| | |
|---|---|
| Natrium Laurylethersulfat: | Texapon N 70 von Firma Cognis, |
| Cocamidopropyl Betain: | Tego-Betain F-50 von Firma Goldschmidt; |
| Sodium Cocoyl Glutamate: | Amisoft ECS 22 SB (pH10) von Firma Ajinomoto; |
| Acrylates Copolymer: | Carbopol Aqua SF-1 Polymer von Firma Noveon; |
| Polyquaternium-7: | Merquat 550L von Firma Ondeo Nalco. |

## Patentansprüche

1. Körperreinigungszubereitung in Form einer Emulsion enthaltend wenigstens 1,8 Gew.-% amphothere oder zwitterionische Tenside und wenigstens 1 Gew.-% Natriumcocoylglutamat und ein vernetztes Copolymer aus Methacrylsäure und C1 bis C4-Alkylacrylat, wobei der Gesamttensidgehalt 6 bis 25 Gew.-% beträgt, **dadurch gekennzeichnet, dass** Öle in Konzentrationen von 5 bis 30 Gew.-%, bevorzugt 7 bis 20 Gew.-%, besonders bevorzugt 9 bis 11 eingesetzt werden.

2. Verwendung von wenigstens 1,8 Gew.-% amphotherer oder zwitterionischer Tenside und wenigstens 1 Gew.-% Natriumcocoylglutamat und einem vernetztem Copolymer aus Methacrylsäure und C1 bis C4-Alkylacrylat bei einem Gesamttensidgehalt von 6 bis 25 Gew.-% zur Verbesserung der Schaummenge von kosmetischen Reinigungsemulsionen.

3. Verwendung von wenigstens 1,8 Gew.-% amphotherer oder zwitterionischer Tenside und wenigstens 1 Gew.-% Natriumcocoylglutamat und einem vernetztem Copolymer aus Methacrylsäure und C1 bis C4-Alkylacrylat bei einem Gesamttensidgehalt von 6 bis 25 Gew.-% zur Verbesserung der Schaumqualität von kosmetischen Reinigungsemulsionen.

4. Zubereitung oder Verwendung nach einem der vorangehenden Patentansprüche,
**dadurch gekennzeichnet, dass** Verhältnis Natriumcocoylglutamat zu amphotheren oder zwitterionischen Tensiden 1,5:1 bis 12:1; bevorzugt 3:1 bis 10:1; besonders bevorzugt 5:1 bis 7:1 beträgt.

5. Zubereitung oder Verwendung nach einem der vorangehenden Patentansprüche,
**dadurch gekennzeichnet, dass** der Methacrylsäureanteil im Copolymer 25 bis 70 Gew.-%, besonders bevorzugt 35 bis 65 Gew.-% beträgt.

6. Zubereitung oder Verwendung nach einem der vorangehenden Patentansprüche,
**dadurch gekennzeichnet, dass** der Anteil an Alkylacrylat im Copolymer 25 bis 75 Gew.-%, besonders bevorzugt 35 bis 65 Gew.-% beträgt.

7. Zubereitung oder Verwendung nach einem der vorangehenden Patentansprüche, **dadurch gekennzeichnet, dass** als Alkylacrylat im Copolymer das Methyl, Ethyl- oder Buylacrylat, besonders bevorzugt das Ethylacrylat eingesetzt wird.

8. Zubereitung oder Verwendung nach einem der vorangehenden Patentansprüche, **dadurch gekennzeichnet, dass** das Copolymer unter Verwendung von 0,03 bis 1 Gew.-% mehrfach ethylenisch ungesättigtem Vernetzungsmittel vernetzt wird.

9. Zubereitung oder Verwendung nach einem der vorangehenden Patentansprüche, **dadurch gekennzeichnet, dass** das Copolymer in Konzentrationen von 1 bis 6 Gew.-%, bevorzugt 2 bis 4 Gew.-%, besonders bevorzugt 2,5 bis 3,5 Gew.-% eingesetzt wird.

10. Zubereitung oder Verwendung nach einem der vorangehenden Patentansprüche, **dadurch gekennzeichnet, dass** Öle in Konzentrationen von 5 bis 30 Gew.-%, bevorzugt 7 bis 20 Gew.-%, besonders bevorzugt 9 bis 11 Gew.-% eingesetzt werden,

11. Zubereitung oder Verwendung nach einem der vorangehenden Patentansprüche, **dadurch gekennzeichnet, dass** Öle in Form von natürlichen Triglyceriden eingesetzt werden, besonders bevorzugt in Anteilen von 10 bis 50 % bezogen auf den Ölanteil, ganz besonders bevorzugt 25 bis 35 %.

12. Zubereitung oder Verwendung nach einem der vorangehenden Patentansprüche, **dadurch gekennzeichnet, dass** die Tröpfchengröße der Emulsion zwischen 500 nm bis 2000 nm, besonders bevorzugt (d(0,5)=1000 nm liegt.

13. Zubereitung oder Verwendung nach einem der vorangehenden Patentansprüche, **dadurch gekennzeichnet, dass** die Tröpfchen der Emulsion in Form von Tröpfchenagglomeraten vorliegen.

14. Zubereitung oder Verwendung nach einem der vorangehenden Patentansprüche, **dadurch gekennzeichnet, dass** die Emulsion keine lamellare Phase aufweist.

15. Zubereitung oder Verwendung nach einem der vorangehenden Patentansprüche, **dadurch gekennzeichnet, dass** ihre Fliessgrenze 3 bis 30 Pa beträgt, gemessen auf einem schubspannungsgesteuerten Rheometer bei 25°C ± 1°C mit 25 mm Platte/Platte Geometrie bei einem Spalt zwischen 0,8 mm und 1,2 mm, wobei strukturschonend befüllt wird, eine geeignete konstante Schubspannungszeitrampe vorgegeben und vor dem Test eine entsprechende Strukturerholungszeit eingehalten und die kritische Schubspannung im Maximum der Fließkurve angegeben wird,

16. Zubereitung oder Verwendung nach einem der vorangehenden Patentansprüche, **dadurch gekennzeichnet, dass** ihre Viskosität 1500 bis 7000 mPas beträgt, gemessen bei 25°C und einer Scherrate von 100 1/s im Rheomat R123, Messsystem 1, Messkörper 1.

17. Zubereitung oder Verwendung nach einem der vorangehenden Patentansprüche, **dadurch gekennzeichnet, dass** sie zusätzlich PQ 7 enthält

18. Zubereitung oder Verwendung nach einem der vorangehenden Patentansprüche, **dadurch gekennzeichnet, dass** sie 0 bis 0,01 Gew.-% Fettalkoholethoxylate enthält.

19. Zubereitung oder Verwendung nach einem der vorangehenden Patentansprüche, **dadurch gekennzeichnet, dass** sie 0 bis 0,01 Gew.-% Fettsäuren enthält.

20. Zubereitung oder Verwendung nach einem der vorangehenden Patentansprüche, **dadurch gekennzeichnet, dass** sie 0 bis 0,01 Gew.-% Polysaccharide enthält.

21. Zubereitung oder Verwendung nach einem der vorangehenden Patentansprüche, **dadurch gekennzeichnet, dass** sie neben dem vernetzten Copolymer aus Methacrylsäure und C1 bis C4-Alkylacrylat keinen weiteren Gelbildner enthält.

22. Zubereitung oder Verwendung nach einem der vorangehenden Patentansprüche, **dadurch gekennzeichnet, dass** sie nicht mehr als ein kationisches Polymer enthält.

## Claims

1. Body cleansing preparation in the form of an emulsion comprising at least 1.8% by weight amphoteric or zwitterionic surfactants and at least 1% by weight sodium cocoyl glutamate and a crosslinked copolymer of methacrylic acid and C1 to C4-alkyl acrylate, wherein the total surfactant content is 6 to 25% by weight, **characterized in that** oils are used at concentrations of 5 to 30% by weight, preferably 7 to 20% by weight, particularly preferably 9 to 11.

2. Use of at least 1.8% by weight of amphoteric or zwitterionic surfactants and at least 1% by weight sodium cocoyl glutamate and a crosslinked copolymer of methacrylic acid and C1 to C4-alkyl acrylate at a total surfactant content of 6 to 25% by weight for improving the amount of foam of cosmetic cleansing emulsions.

3. Use of at least 1.8% by weight of amphoteric or zwitterionic surfactants and at least 1% by weight sodium cocoyl glutamate and a crosslinked copolymer of methacrylic acid and C1 to C4-alkyl acrylate at a total surfactant content of 6 to 25% by weight for improving the foam quality of cosmetic cleansing emulsions.

4. Preparation or use according to any of the preceding patent claims, **characterized in that** the ratio of sodium cocoyl glutamate to amphoteric or zwitterionic surfactants is from 1.5:1 to 12:1, preferably from 3:1 to 10:1, particularly preferably from 5:1 to 7:1.

5. Preparation or use according to any of the preceding patent claims, **characterized in that** the proportion of methacrylic acid in the copolymer is 25 to 70% by weight, particularly preferably 35 to 65% by weight.

6. Preparation or use according to any of the preceding patent claims, **characterized in that** the proportion of alkyl acrylate in the copolymer is 25 to 75% by weight, particularly preferably 35 to 65% by weight.

7. Preparation or use according to any of the preceding patent claims, **characterized in that** the alkyl acrylate used in the copolymer is methyl, ethyl or butyl acrylate, particularly preferably ethyl acrylate.

8. Preparation or use according to any of the preceding patent claims, **characterized in that** the copolymer is crosslinked using 0.03 to 1% by weight polyethylenically unsaturated crosslinking agent.

9. Preparation or use according to any of the preceding patent claims, **characterized in that** the copolymer is used at concentrations of 1 to 6% by weight, preferably 2 to 4% by weight, particularly preferably 2.5 to 3.5% by weight.

10. Preparation or use according to any of the preceding patent claims, **characterized in that** oils are used at concentrations of 5 to 30% by weight, preferably 7 to 20% by weight, particularly preferably 9 to 11% by weight.

11. Preparation or use according to any of the preceding patent claims, **characterized in that** oils are used in the form of natural triglycerides, particularly preferably in proportions of 10 to 50%, based on the oil proportion, especially preferably 25 to 35%.

12. Preparation or use according to any of the preceding patent claims, **characterized in that** the droplet size of the emulsion is between 500 nm to 2000 nm, particularly preferably d(0.5) = 1000 nm.

13. Preparation or use according to any of the preceding patent claims, **characterized in that** the droplets of the emulsion are in the form of droplet agglomerates.

14. Preparation or use according to any of the preceding patent claims, **characterized in that** the emulsion has no lamellar phase.

15. Preparation or use according to any of the preceding patent claims, **characterized in that** its yield point is 3 to 30 Pa, measured on a shear stress-controlled rheometer at 25°C ± 1°C with 25 mm plate/plate geometry at a gap between 0.8 mm and 1.2 mm, wherein charging is carried out in a structure-preserving manner, a suitable constant shear stress gradient is pre-defined and, prior to the test, a corresponding structure recovery time is observed and the critical shear stress at the maximum of the flow curve is specified.

16. Preparation or use according to any of the preceding patent claims, **characterized in that** its viscosity is 1500 to 7000 mPas, measured at 25°C and a shear rate of 100 1/s on a Rheomat R123, measuring system 1, measuring body 1.

17. Preparation or use according to any of the preceding patent claims, **characterized in that** it additionally comprises PQ 7.

18. Preparation or use according to any of the preceding patent claims, **characterized in that** it comprises 0 to 0.1% by weight fatty alcohol ethoxylates.

19. Preparation or use according to any of the preceding patent claims, **characterized in that** it comprises 0 to 0.01% by weight fatty acids.

20. Preparation or use according to any of the preceding patent claims, **characterized in that** it comprises 0 to 0.01% by weight polysaccharides.

21. Preparation or use according to any of the preceding patent claims, **characterized in that** it does not comprise any further gel formers in addition to the crosslinked copolymer of methacrylic acid and C1 to C4-alkyl acrylate.

22. Preparation or use according to any of the preceding claims, **characterized in that** it does not comprise more than one cationic polymer.

## Revendications

1. Préparation de nettoyage corporel sous forme d'une émulsion contenant au moins 1,8 % en poids de tensioactifs amphotères ou zwitterioniques et au moins 1 % en poids de glutamate de cocoyle sodique et un copolymère réticulé composé d'acide méthacrylique et d'un acrylate d'alkyle en C1 à C4, la teneur totale en tensioactif étant de 6 à 25 % en poids, **caractérisée en ce que** des huiles sont utilisées en des concentrations de 5 à 30 % en poids, préférablement 7 à 20 % en poids, particulièrement préférablement 9 à 11.

2. Utilisation d'au moins 1,8 % en poids de tensioactifs amphotères ou zwitterioniques et d'au moins 1 % en poids de glutamate de cocoyle sodique et d'un copolymère réticulé composé d'acide méthacrylique et d'un acrylate d'alkyle en C1 à C4 pour une teneur totale en tensioactif de 6 à 25 % en poids pour l'amélioration de la quantité de mousse d'émulsions de nettoyage cosmétiques.

3. Utilisation d'au moins 1,8 % en poids de tensioactifs amphotères ou zwitterioniques et d'au moins 1 % en poids de glutamate de cocoyle sodique et d'un copolymère réticulé composé d'acide méthacrylique et d'un acrylate d'alkyle en C1 à C4 pour une teneur totale en tensioactif de 6 à 25 % en poids pour l'amélioration de la qualité de la mousse d'émulsions de nettoyage cosmétiques.

4. Préparation ou utilisation selon l'une quelconque des revendications de brevet précédentes, **caractérisée en ce que** le rapport de glutamate de cocoyle sodique sur les tensioactifs amphotères ou zwitterioniques est de 1,5 : 1 à 12 : 1 ; préférablement 3 : 1 à 10 : 1 ; particulièrement préférablement 5 : 1 à 7: 1.

5. Préparation ou utilisation selon l'une quelconque des revendications de brevet précédentes, **caractérisée en ce que** la proportion d'acide méthacrylique dans le copolymère est de 25 à 70 % en poids, particulièrement préférablement 35 à 65 % en poids.

6. Préparation ou utilisation selon l'une quelconque des revendications de brevet précédentes, **caractérisée en ce que** la proportion d'acrylate d'alkyle dans le copolymère est de 25 à 75 % en poids, particulièrement préférablement 35 à 65 % en poids.

7. Préparation ou utilisation selon l'une quelconque des revendications de brevet précédentes, **caractérisée en ce qu'**en tant qu'acrylate d'alkyle dans le copolymère, l'acrylate de méthyle, d'éthyle ou de butyle, particulièrement préférablement l'acrylate d'éthyle est utilisé.

8. Préparation ou utilisation selon l'une quelconque des revendications de brevet précédentes, **caractérisée en ce que** le copolymère est réticulé en utilisant de 0,03 à 1 % en poids d'agent de réticulation plusieurs fois éthyléniquement insaturé.

9. Préparation ou utilisation selon l'une quelconque des revendications de brevet précédentes, **caractérisée en ce que** le copolymère est utilisé en des concentrations de 1 à 6 % en poids, préférablement de 2 à 4 % en poids, particulièrement préférablement de 2,5 à 3,5 % en poids.

10. Préparation ou utilisation selon l'une quelconque des revendications de brevet précédentes, **caractérisée en ce que** des huiles sont utilisées en des concentrations de 5 à 30 % en poids, préférablement 7 à 20 % en poids, particulièrement préférablement 9 à 11 % en poids.

11. Préparation ou utilisation selon l'une quelconque des revendications de brevet précédentes, **caractérisée en ce que** des huiles sous forme de triglycérides naturels sont utilisées, particulièrement préférablement en des proportions de 10 à 50 % par rapport à la proportion d'huile, tout particulièrement préférablement 25 à 35 %.

12. Préparation ou utilisation selon l'une quelconque des revendications de brevet précédentes, **caractérisée en ce que** la taille de gouttelettes de l'émulsion se situe entre 500 nm et 2 000 nm, particulièrement préférablement d(0,5) = 1 000 nm.

13. Préparation ou utilisation selon l'une quelconque des revendications de brevet précédentes, **caractérisée en ce que** les gouttelettes de l'émulsion sont présentes sous forme d'agglomérats de gouttelettes.

14. Préparation ou utilisation selon l'une quelconque des revendications de brevet précédentes, **caractérisée en ce que** l'émulsion ne présente aucune phase lamellaire.

15. Préparation ou utilisation selon l'une quelconque des revendications de brevet précédentes, **caractérisée en ce que** sa limite d'écoulement est de 3 à 30 Pa, mesurée sur un rhéomètre commandé par contrainte de poussée à 25 °C ± 1 °C avec une géométrie plaque/plaque de 25 mm avec une fente comprise entre 0,8 mm et 1,2 mm, le remplissage étant effectué avec ménagement de la structure, une pente en fonction du temps de contrainte de poussée constante appropriée étant prédéfinie et, avant l'essai, un temps de récupération de structure correspondant étant maintenu et la contrainte de poussée critique étant indiquée par le maximum de la courbe d'écoulement.

16. Préparation ou utilisation selon l'une quelconque des revendications de brevet précédentes, **caractérisée en ce que** sa viscosité est de 1 500 à 7 000 mPas, mesurée à 25 °C et à une vitesse de cisaillement de 100 1/s dans un Rheomat R123, système de mesure 1, corps de mesure 1.

17. Préparation ou utilisation selon l'une quelconque des revendications de brevet précédentes, **caractérisée en ce qu'**elle contient de plus du PQ 7.

18. Préparation ou utilisation selon l'une quelconque des revendications de brevet précédentes, **caractérisée en ce qu'**elle contient 0 à 0,01 % en poids d'éthoxylates d'alcools gras.

19. Préparation ou utilisation selon l'une quelconque des revendications de brevet précédentes, **caractérisée en ce qu'**elle contient 0 à 0,01 % en poids d'acides gras.

20. Préparation ou utilisation selon l'une quelconque des revendications de brevet précédentes, **caractérisée en ce qu'**elle contient 0 à 0,01 % en poids de polysaccharides.

21. Préparation ou utilisation selon l'une quelconque des revendications de brevet précédentes, **caractérisée en ce qu'**elle ne contient, outre le copolymère réticulé composé d'acide méthacrylique et d'un acrylate d'alkyle en C1 à C4, aucun autre agent de formation de gel.

22. Préparation ou utilisation selon l'une quelconque des revendications de brevet précédentes, **caractérisée en ce qu'**elle ne contient pas plus d'un polymère cationique.
